# EUROPEAN PATENT APPLICATION

(11) **EP 2 233 064 A1**
(43) Date of publication of application: **29.09.2010**
(21) Application number: 10250548.4
(22) Date of filing: 23.03.2010
(51) Int. Cl.: A61B 1/313, A61B 17/34, A61B 17/00

(54) **Three piece elastic disk**

(30) Priority: 24.03.2009 US 162826 P; 04.03.2010 US 717300
(71) Applicant: Tyco Healthcare Group LP, North Haven, CT 06473 (US)
(72) Inventor: Battles, Christopher A., Seymour, CT 06483 (US)
(74) Representative: Soames, Candida Jane

(57) **Abstract**

A seal device to create an airtight seal around a variety of laparoscopic instruments passed through laparoscopic ports. The seal device comprises a plurality of overlapping elastic disks with each disk defining an aperture and configured to accommodate an instrument. The elasticity of each disk maintains a seal around the instrument as the instrument is manipulated.

## Description

The present application claims the benefit of and priority to U.S. Provisional Application Serial No. 61/162,826 filed on March 24, 2009, the entire contents of which are incorporated herein by reference.

### BACKGROUND

### 1. Technical Field

The present disclosure relates to an apparatus for a seal device to create an airtight seal around a variety of laparoscopic instruments passed through laparoscopic ports.

### 2. Background of Related Art

Minimally invasive surgical procedures, such as laparoscopic surgery, result in reduced trauma for a patient than would an equivalent open procedure. In this procedure, narrow hollow tubes called cannula are inserted into small incisions in the skin made by a trocar. Elongated surgical instruments are introduced through the cannula.

Since the body cavity is often insufflated with carbon dioxide to create a working space by separating the cavity wall from the internal organs therein, care must be taken to prevent the unacceptable entry or exit of gases or fluids. Various seals have been designed for this purpose. As instruments are inserted and manipulated within the seal, the integrity of the seal is at risk.

In large measure, the integrity of the seal is dependent upon the relative dimensions of the instrument shaft and the seal opening. If the contact pressure between the instrument and the seal opening is too little, insufflation pressure may not be maintained. On the other hand, if the contact pressure is too great there will be difficulty inserting, removing, and/or manipulating the instrument.

The known seals are deficient in numerous ways, including an inability to accommodate instrumentation of a wide range of sizes and an inability to preserve the integrity of the seal during the procedure.

### SUMMARY

The present disclosure describes a seal device to create an airtight seal around a variety of laparoscopic instruments passed through laparoscopic ports. The seal device comprises a plurality of overlapping elastic disks, each disk defining an aperture and configured to accommodate an instrument. The elasticity of each disk maintains a seal around the instrument as the instrument is manipulated.

The invention may be described by reference to the following numbered paragraphs:-

A seal device comprising: a plurality of elastic disks, each disk defining an aperture, at least a portion of the aperture of each elastic disk being offset or misaligned relative to the apertures of the other elastic disk.

The seal device of paragraph [0009], wherein, in a relaxed position, the radially innermost edge of each aperture is located substantially at or near the longitudinal axis of the seal device, so as to substantially prevent the flow of fluid through the seal device.

The seal device of paragraph [0009] or [0010], wherein, in an operative position, e.g., having an instrument inserted therethrough, the elastic material of each elastic disk is caused to stretch so as to accommodate the instrument.

The seal device of paragraph [0011], wherein a portion of each one of the apertures, such portion being adjacent to a radially innermost edge of the aperture, collectively seal about the instrument.

The seal device of any preceding paragraph, wherein each disk defines an aperture of a substantially elliptical shape with one focus at the central axis of the disks.

The seal device of any of paragraph [0009] to [0012], wherein the disks are arranged such that the apertures defined by the disks are located radially along the central axis and are equidistant between each other.

The seal device of any preceding paragraph, wherein the aperture is formed by extruding a cut in each disk of a first semicircle alongside a rectangle alongside a second semicircle, the first semicircle having its center point defined by the central axis of the disk.

### BRIEF DESCRIPTION OF THE DRAWINGS

By way of description only, embodiments of the disclosure will be described with reference to the accompanying drawings, in which:

Fig. 1 is a top view of a seal device in an initial state according to the present disclosure;

Fig. 2 is a top view of the seal device of Fig. 1 in a second state for accommodating an instrument;

Fig. 3 is a top view of the seal device of Fig. 2 with the instrument moved off-center; and

Fig. 4 is a front view of the seal device of Fig. 1.

### DETAILED DESCRIPTION OF THE EMBODIMENTS

Particular embodiments of the present disclosure will be described herein with reference to the accompanying drawings. In the following description, well-known functions or constructions are not described in detail to avoid obscuring the present disclosure in unnecessary detail.

The seal device 100 allows the introduction and manipulation of a variety of instruments adapted for insertion through a trocar or cannula assembly while preserving the atmospheric integrity of the body cavity from gas or fluid leakage. Examples of instrumentation include clip appliers, graspers, dissectors, retractors, staplers, laser probes, photographic devices, endoscopes and laparoscopes, tubes, and the like. Such instruments will be collectively referred to herein as "instruments or instrumentation".

The seal device 100 is adapted for use with a trocar assembly, including an obturator and a cannula, and is utilized for minimally invasive, such as endoscopic or laparoscopic procedures. The seal device 100 cooperates with the obturator or other instruments extending through the cannula to form a seal around the outer surface of the instrument and inhibit the passage of fluids or gases through the body cavity and trocar assembly, i.e., across the seal boundary.

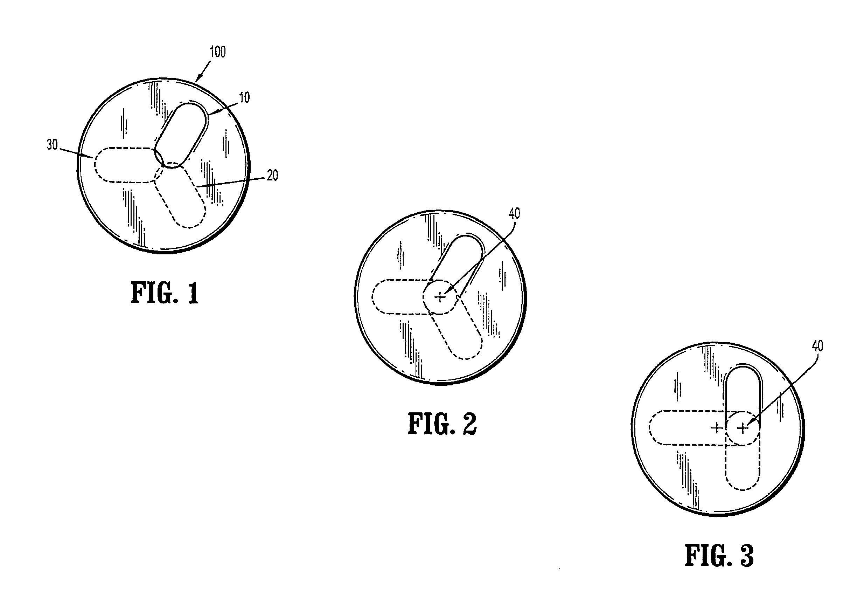

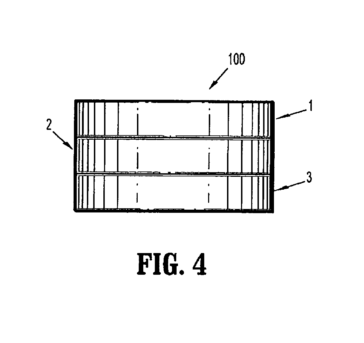

The seal device 100 is capable of accommodating a surgical instrument of a variety of dimensions. Fig. 1 shows the seal device 100 as it would look in its initial state without an instrument inserted therethrough. Three disks, 1, 2, and 3 are placed one on top of the other, as shown in Fig. 4. Each aperture 10, 20, and 30 is located radially along the central axis of the disk and equidistant from each other. The overlapping disks 1, 2, and 3 together define an aperture 40 for the entire device 100 through which an instrument can be inserted. Aperture 40 elastically expands to allow for the passage of instruments inserted therein. While the illustrated embodiment uses three disks, it is contemplated that other quantities of disks may be used.

As an instrument is inserted through the aperture 40, defined by the three apertures of the individual disks, 10, 20, and 30, each disk 1, 2, and 3 stretches to accommodate the instrument as shown in Fig. 2. The elasticity of the individual disks 10, 20, and 30 ensures that a seal is maintained around the instrument inserted in aperture 40, which stretches as necessary to accommodate the instrument diameter. Movement of the instrument either stretches or relaxes the disks 1, 2, and 3, thereby changing the size of the aperture 40. This creates an elastic force around the surgical instrument and helps minimize fluid and gas leakage between the seal device 100 and the instruments.

Balance between a tight seal and ease of instrument manipulation can be achieved by selecting appropriately sized instrument shafts relative to the relaxed apertures 10, 20, and 30 of disks 1, 2, and 3. Frictional resistance between the instrument and the disks 1, 2, and 3 can be reduced by selecting instruments having shaft sizes only slightly larger than the relaxed diameters of the apertures 10, 20, and 30 of disks 1, 2, and 3.

The seal device 100 can be made by any number of conventional techniques, including but not limited to, liquid injection molding, plastic injection molding, and/or transfer molding. Furthermore, the disks, 1, 2, and 3, can be made from a variety of materials having elastic or compliant properties, including, but not limited to elastomers such as cellular rubbers, natural rubbers, and/or synthetic rubbers. Alternatively, the material may comprise a viscoelastic gel.

In another embodiment, a flexible casing containing a predetermined quantity of fluid may be used. Optionally, a fabric material, such as SPANDEX containing a mixture of LYCRA and NYLON can be superposed over the seal device 100 to minimize the potential of piercing, penetrating or tearing of the resilient material by the instrument. The combination of a resilient material and a fabric material would resist both the deformation of the aperture defined by the disks 1, 2, and 3 as well as damage to the disks 1, 2, and 3. The selected material can also be coated or impregnated with a therapeutic agent or material, such as an oligodynamic metal or an antimicrobial medium.

It will be understood by those skilled in the art that various modifications and changes in form and detail may be made therein without departing from the scope and spirit of the present disclosure. Accordingly, modifications such as those suggested above, but not limited thereto, are to be considered within the scope of the disclosure. Therefore, the above description should not be construed as limited to the disclosed embodiments. Other embodiments within the scope and spirit of the present invention will appear to those skilled in the art.

## Claims

1. A seal device comprising: a plurality of elastic disks, each disk defining an aperture, at least a portion of the aperture of each elastic disk being offset or misaligned relative to the apertures of the other elastic disk.

2. The seal device of claim 1, wherein, in a relaxed position, the radially innermost edge of each aperture is located substantially at or near the longitudinal axis of the seal device, so as to substantially prevent the flow of fluid through the seal device.

3. The seal device of claim 1 or claim 2, wherein, in an operative position, e.g., having an instrument inserted therethrough, the elastic material of each elastic disk is caused to stretch so as to accommodate the instrument.

4. The seal device of claim 3, wherein a portion of each one of the apertures, such portion being adjacent to a radially innermost edge of the aperture, collectively seal about the instrument.

5. The seal device of any preceding claim, wherein each disk defines an aperture of a substantially elliptical shape with one focus at the central axis of the disks.

6. The seal device of any of claims 1 to 4, wherein the disks are arranged such that the apertures defined by the disks are located radially along the central axis and are equidistant between each other.

7. The seal device of any preceding claim, wherein the aperture is formed by extruding a cut in each disk of a first semicircle alongside a rectangle alongside a second semicircle, the first semicircle having its center point defined by the central axis of the disk.
